# EUROPEAN PATENT SPECIFICATION

(11) **EP 0 650 707 B1**
(45) Date of publication and mention of the grant of the patent: **13.11.1996**
(21) Application number: 94307798.2
(22) Date of filing: 25.10.1994
(51) Int. Cl.: A61F 2/46

(54) **Apparatus for implanting an acetabular cup**
Vorrichtung zum Implantieren einer Hüftgelenkspfanne
Appareil pour l'implantation d'une cupule acétabulaire

(30) Priority: 29.10.1993 GB 9322327
(43) Date of publication of application: 03.05.1995
(73) Proprietor: HOWMEDICA INTERNATIONAL INC., Shannon Co. Clare (IE)
(72) Inventor: Ashby, Alan Miles, Maidenhead, Berkshire, SL6 4LD (GB); Orton, Marcus Andrew, Thame, Oxfordshire, 0X9 3XW (GB)
(74) Representative: Bridge-Butler, Alan James

(56) References cited:
- EP-A- 0 535 973
- US-A- 5 116 339

## Description

This invention relates to apparatus for implanting an acetabular cup which is to be held in place by cement.

It is known, for example, from the Charnley (type) ogee Flanged Cup to provide a flange on the cup to pressurise the cement but this only occurs as the cup approaches its final position in the acetabulum. The flexible flange touches the acetabular margin tending to occlude cement flow.

Another device for pressurising cement is known as the Exeter Acetabular Pressuriser. In this arrangement a water filled seal mounted on the end of a handle is used after the acetabulum has been filled with cement but before the cup is introduced, the seal covers the acetabulum and the surgeon pushes on the handle thereby forcing the doughy cement in the holes, ridges and trabeculae. When the instrument is removed and pressure is released blood flow can squeeze between the cement and the bone.

EP 0 535 973 which is used as a basis for the preamble of claim 1 describes a surgical instrument for inserting acetabular cups and which may also be used to maintain pressure on the cup while the bone cement is setting. In the construction described the cup to be inserted is placed on a hemispherical button and pressure is maintained through a trigger mechanism. There is however no means for guiding or aligning the cup into the acetabular socket or for locating it at a predetermined position therein.

It is also known to provide cup positioning instruments with long pointers (vertical, horizontal and at right angles to the patient's centre line of body symmetry) to enable the surgeon to control cup orientation reasonably well provided the pelvis does not move. Linear positioning of the cup is determined by eye in a medio-lateral and anterio-posterior sense. If the natural acetabulum is well formed a by-eye judgement can be acceptable but many acetabulae are deformed or eroded in which case by-eye judgement can be confused.

A further method is to use what are usually referred to as acetabular spacers. These can be lugs fixed to the outside of the acetabular cup or can be press studs pushed into the bone base of the natural acetabulum. These prevent the cup from being pushed too deeply into the acetabular socket and can control medio-lateral and anterio-posterior positioning. However, they force some degree of centralisation of the cup. If the surgeon chooses to place the cup eccentrically to make use of a particular area of solid bone support, acetabular spacers do not help.

The present invention is intended to provide apparatus for overcoming some of the difficulties referred to above, to allow pressurisation of the cement so that good holding is obtained and to provide a method of operation which will ensure accurate location of the cup in the socket.

According to the present invention apparatus for implanting an acetabular cup is characterised by a loading tube adapted for alignment with an acetabular socket the bore of said tube being dimensioned to receive a suitably dimensioned acetabular cup as a sliding fit to guide it into said socket, an inserter for pushing said acetabular cup down said tube, and stop means which act on said inserter or an attachment thereto to limit movement of said inserter down said tube to a predetermined position therein.

In a preferred construction said inserter acts on the rim of the cup, and thus the inserter can be in the form of a piston which is a sliding fit within the bore of the tube.

The stop means can be provided on part of the bore of said tube and act on the inner end of the inserter, this construction being particularly applicable when a flanged socket is used, the socket being provided with a gap in the flange.

In an alternative construction stop means can be provided on the inserter which engage an abutment on said tube located at a position spaced away from the end of the tube which is intended to be adjacent the acetabular socket when in use. This construction can be used with sockets having a flange or a plain upper rim.

If desired the diameter of the tube can be adjustable

The tube may also be provided with a flexible skirt.

With this construction it is possible to either fill the acetabulum with doughy cement and then reposition the skirt and tube or the tube on the bone using markers to ensure that a previously chosen position and orientation are re-established, or the cement can be injected, for example by a cement gun, through the tube. The surgeon can then push the cup into the cement with the inserter to create a pressure and ensure that the cement fills all the openings in the socket. If the flexible skirt assembly is used it can be secured around the rim of the socket preventing the escape of cement and allowing it to be pressurised.

The flexible skirt can be held in place by means of screws, pins and/or staples, or finger pressure alone.

The method also includes allowing some of the pressurised cement to escape from the socket and through a controlled orifice in the skirt.

With this arrangement the tube can be integral with the flexible skirt or it can be detached therefrom.

Preferably the flexible skirt is made from a synthetic plastics material and if it is to be left in place could be polyethylene, polypropylene, polyurethane, or even a resorbable material for example polyglycolic acid, or polylactic acid.

In order to locate the cup the surgeon can control the release of cement through the orifice in the skirt, he can push the cup with the inserter thereby feeling the desired level of cement pressure. If he is not able to reach the desired predetermined position when the stop means are activated he can release the pressure on the cement, allowing a little more cement to escape whilst the cup progresses further down the inside of the tube.

An advantage of the invention is that no spacers are required which tend to cause discontinuities in the cement mantle and which in turn increases the risk of the cement cracking. The arrangement also enables the surgeon to accurately locate the position and attitude of the cup.

Means can also be included for positioning and orientating the tube or the tube and skirt on the bone into which the cup is to be implanted.

Preferably said stop means can be adjusted, removed or overridden to allow said inserter to be moved further down said tube beyond said predetermined position. Thus in one embodiment according to the invention said stop means is provided by a resiliently biased catch which can engage in a slot provided in the wall of said tube and which can move to an inoperative position to allow said inserter to pass beyond a predetermined position.

The invention can be performed in various ways and various embodiments will now be described by way of example and with reference to the accompanying drawings in which :
Figure 1 is a diagrammatic cross-sectional view of one example of the apparatus showing the method of operation;
Figure 2 shows a construction of a variable diameter tube;
Figure 3 is a diagrammatic side view of another form of apparatus according to the invention;
Figure 4 is a diagrammatic side view of another form of the apparatus which would be supplied as a series of designs with a wedge or sloping orientation to accommodate deformed acetabular socket rims;
Figures 5, 6 and 7 show how a step can be machined around the rim of the acetabulum showing how the apparatus can be used if the bony rim is eroded;
Figure 8 shows in diagram form how an orientation guide can be provided;
Figures 9, 10 and 11 show three steps in performing the method and using the apparatus when using a trial cup;
Figure 12 is a diagrammatic side view of apparatus similar to that shown in Figure 3 but without the same use of a skirt;
Figures 13, 14, 15 and 16 are isometric views of alternative forms of inserter;
Figure 17 is an isometric view of another tube and inserter construction;
Figure 18 is a cross-sectional side elevation through the construction shown in Figure 17;
Figure 19 is an isometric view from below of part of the inserter shown in Figure 18; and,
Figure 20 is a plan view from below of the construction shown in Figure 17.

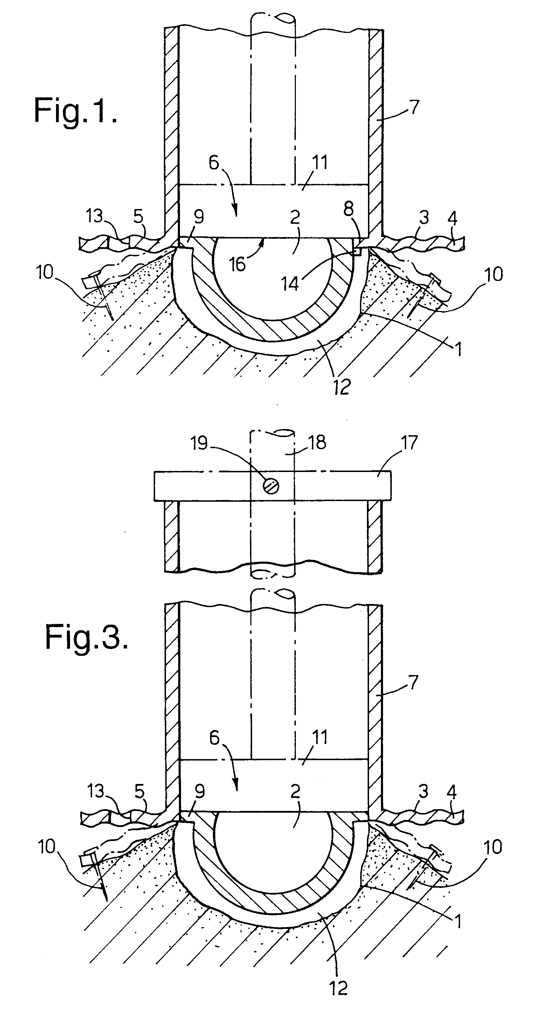

Figure 1 shows a general form of the apparatus, an acetabular socket being indicated by reference numeral 1 into which an acetabular cup 2 is to be fitted.

In order to fit the cup into the socket 1 in which it is to be held by cement in this embodiment a flexible skirt assembly 3 is utilised. This skirt assembly 3 is made as an integral unit from a synthetic plastics material, for example polyethylene, polypropylene, polyurethane, or even a resorbable material, for example polyglycolic acid, or polylactic acid. This assembly comprises a flexible skirt 4 which is shown with corrugations 5. The skirt has a central opening 6 and is provided with a loading tube 7 the inner bore of which is substantially the same diameter as the opening 6. The lower end of the tube is formed with an inwardly projecting abutment 8. The acetabular cup 2 has an annular flange 9 having a gap 14 which is dimensioned to allow the abutment 8 to pass through it and is a sliding fit in the tube 7.

To implant the cup 2 the acetabular socket 1 is first prepared in the usual way. The flexible skirt assembly 3 is now placed in the position shown in full lines in the drawing and the skirt 4 is bent and fixed to the bone around the acetabular opening by pins, screws or staples indicated by reference numeral 10. The deformed position of the skirt when in its secured position is indicated in broken lines.

If desired the skirt 4 can be preformed to the required shape. The relative positioning on to the acetabular bone can be controlled by positioning guides, with or without fixing.

Prior to fastening it in position however the position and orientation of the tube 7 is determined during the early preparatory stages of the operation and once this has been determined the skirt 4 can be trimmed if necessary and fastened in position. It will be seen that on the right hand side of the drawing the width of the skirt 4 has been trimmed down to assist in location.

The prepared acetabulum is now filled with cement through the tube 7, the cement being pushed into place by a manually operated inserter in the form of a piston 11 which is a sliding fit in the tube 7 and acts on the rim of the cup 2. The piston is removed when the cement is partially set and in an adequately doughy stage. The cup 2 is immediately inserted down the tube 7 using the same or an alternative inserter and thus the cement is pressurised from the moment the cup touches it. The gap 14 in the cup flange 9 is aligned with the abutment 8. In the arrangement shown the piston 11 is used to push the cup into place the piston descending until a peripheral portion of the edge of its front face 16 engages abutment 8 thereby preventing the surgeon from pushing the cup in too far and thus locating it at a predetermined position.

As will be seen from the drawing no spacers are required in the cement mantle which now surround the cup and which is indicated by reference numeral 12.

A cement flow release arrangement is provided by the provision of an orifice 13 in the skirt 4. The hole in the skirt is occluded, for example by the surgeon's thumb, to allow the pressure to build up and then allow excess cement to escape.

With the cup 2 in place and the cement fully cured the tube is cut away and the remainder of the skirt left implanted.

Alternatively the tube and skirt can be removed.

In the arrangement described above the tube 7 is integral with the skirt 4 but if desired it could be made as a separate removable item.

The use of the skirt 4 also enables the position and orientation of the cup 2 to be determined prior to the actual insertion and to allow the surgeon to place it precisely where he wants it in the acetabular socket.

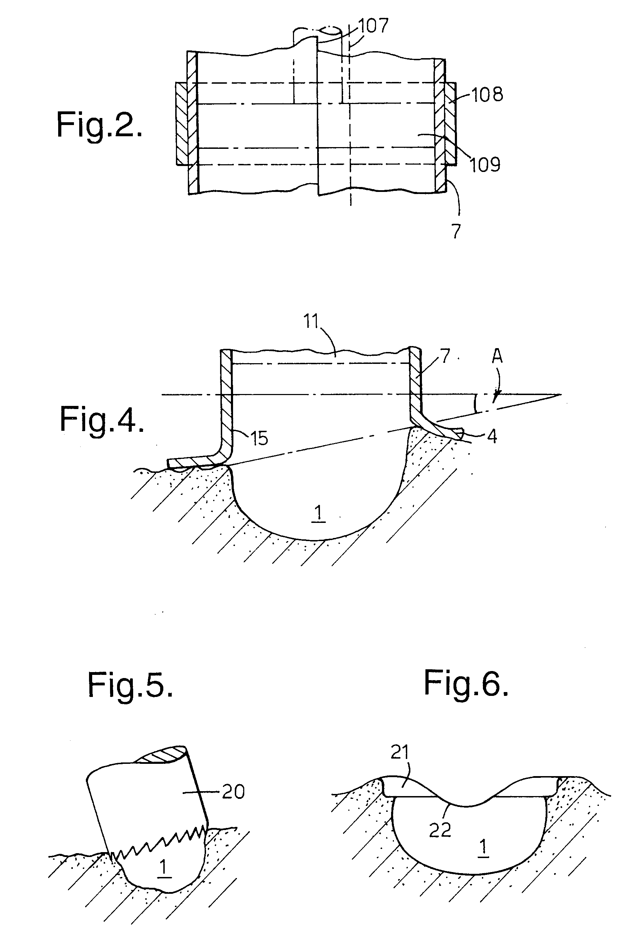

It will of course be necessary to provide skirts in a choice of internal shapes and sizes to suit the chosen cup shape and size. The tube 7 can also be provided in a similar choice of diameters but an adjustable tube could be set to the required diameter and fixed with a circumferential strap, an inserter, for example in the form of a piston of appropriate dimensions being employed. Such a construction is shown in Figure 2. The tube 7 is split and overlaps at its edges as indicated by reference numeral 107 and is held at the required diameter by a strap 108, a larger size piston 109 is also shown.

Figure 3 shows another construction in which the cup can be accurately located in the socket and the same reference numerals are used to indicate similar parts to those shown in Figure 1. With this construction however stop means are provided in the form of an adjustable disc 17 carried on a piston rod 18 of the piston 11. The disc 17 is held by a grub screw 19 passing through the disc and bearing against the piston rod 18. The screw 19 cam be released to allow adjustment of the disc 17 on the rod 18.

As will be seen from Figure 3 a cup 2 with a full projecting annular flange 9 is used but the position of the cup in the socket 12 is located by adjustment of the disc 17 which acts to limit the movement of the piston 11.

As with the construction shown in Figure 1 the tube 7 and the flange 3 can be removed completely after the cement has cured or the tube can be cut away and the flange 3 left in place.

If desired an adjustable loading tube as shown in Figure 2 can be used.

In order to assist in removing the tube 7 the screw 19 can be released thus allowing the piston 11 to be pushed further and with applied upward pressure on the tube it will release from the cup 2.

Figure 4 shows a construction of combined skirt 4 and tube 7 which can be used for deformed/eroded acetabular rims. The tubes and skirts can be supplied as a series of designs with a wedge or slope orientation to accommodate different angles of deformity, the slope angle in Figure 4 is indicated by reference numeral "A"

As will be seen in Figure 4 in this construction the length of the lower end of the inner bore wall 15 varies about the diameter to provide the angled effect shown. A piston 11 and stop means are used as shown in Figure 3, but if desired the stop means of Figure 1 could be utilised, a suitable abutment being provided on the inner bore wall. An adjustable tube as shown in Figure 2 can be used if desired.

The series of designs could of course also be provided in different sizes as well as in wedge/ramp angles.

This construction can also be provided by arranging for the skirt 4 and lower part of the tube wall 15 to be deformable so that the desired shape and wedge/ramp angle is formed by the operating surgeon.

The natural bony rim of the acetabulum is not a uniplanar circular feature and if desired the surgeon can machine a step around the rim to receive a sealing feature or the like.

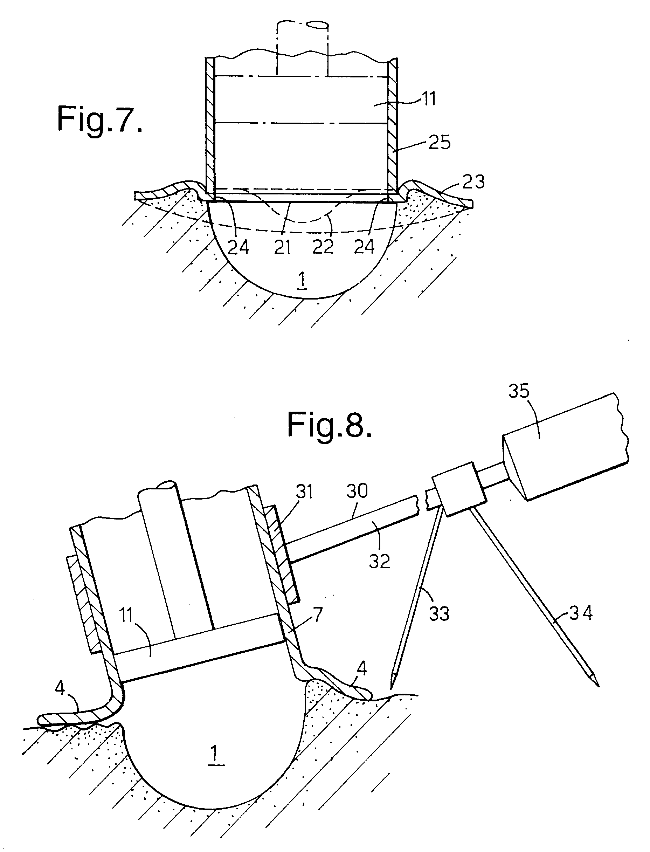

Figures 5 and 6 show how a trepanning cutter 20 is used to machine the rim depth to provide a step 21 inside the natural bony rim. The natural depression in the rim of the acetabulum is indicated by reference numeral 22. Figure 7 shows how a skirt 23 can be used which provides an inwardly directed ledge 24 when placed on the step 21. The skirt extends upwardly and then outwardly and a removable tube 25 is placed in position on it. As will be seen from Figure 7 the deformable skirt 23 is large enough to extend around and cover the natural dip 22 and prevent loss of cement when the piston 11 is operated. Once again the stop means can be as shown in Figure 1 or Figure 3, and an adjustable tube can be used if desired.

The tube 25 can be arranged so that it is a relative push fit into the walls of the step 21 where they are covered by the upstanding portion of the skirt 23.

It will be appreciated that a fixed tube 25 fixed to the skirts 4 could be used if desired.

A further advantage of machining the step 21 is that if it is cut to a predetermined depth it provides a locating means for referencing the cup depth position. Thus with the end of the tube 25 in position on the skirt 23 which then provides the ledge 24 a specific dimension can be obtained between the stopped position of the piston 11 and the step 21 to provide a cup depth position to ensure that there is an adequate cement mantle all around the cup and its flange 9.

In present surgical techniques positioning and orientating an acetabular cup is sometimes achieved using a cup introducer/inserter with two alignment pointers, one of which points at 90° across the centre line (axis of symmetry) of the patient and the other points vertically. This controls only orientation. Position and depth of insertion are chosen by the surgeon by eye.

In the construction shown in Figure 8 the same reference numerals are used to indicate similar parts as shown in Figures 1 and 2 and an orientation guide 30 is provided which has a carrier 31 which surrounds the tube 7 and a long handle 32. The handle 32 carries adjustable alignment pointers 33, 34.

The flexible skirt 4 can be steered by the handheld alignment and orientation guide 30 which is provided with a handle grip 35. The skirt is flexible enough to cope with deformed bony rims around the natural acetabulum and the alignment and orientation guide keeps everything in position and maintains the skirt 4 tight against the bone.

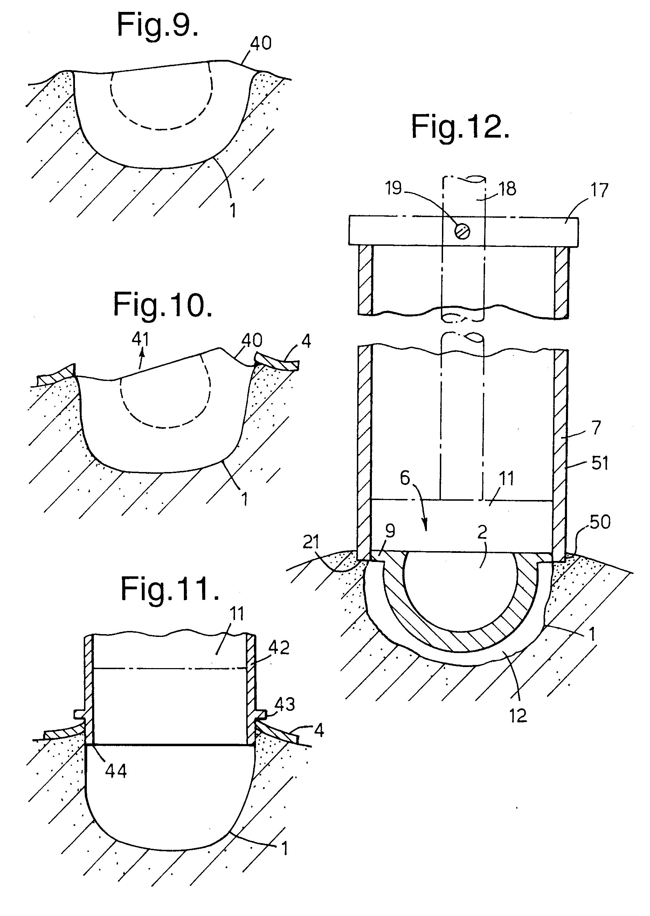

Figures 9, 10 and 11 show an alternative method of employing the apparatus. An important task in this type of operation is to define the desired position and orientation of the cup. This is usually done by putting a trial or dummy implant in place. Theoretically it could be determined before the operation using X-rays.

When using the present invention it is necessary to place the tube and skirt such that the cup will eventually be positioned and tilted as required. The tube and skirt might be placed over a trial/dummy cup fixed and then the trial/dummy removed. Alternatively the surgeon might remove the trial/dummy first and immediately fit the tube and skirt on the basis that his by-eye judgement of position and orientation is good enough.

Figure 9 shows a prepared acetabulum 1 into which a trial acetabular cup 40 has been fitted. At this stage the surgeon chooses the position of centre and orientation.

Figure 10 shows how the surgeon can now fit the flexible skirt 4 around the trial cup 40 and the trial cup can then be removed, as indicated by arrow 41, without disturbing the skirt 4.

After the trial cup 40 has been removed, leaving the skirt in place, there is space for the loading tube (with or without an internally projecting abutment 8 according to the apparatus used) to be assembled to the skirt 4, for example as shown in Figure 11, and for the implant equivalent to the trial cup 40 but necessarily smaller in size to be put in position.

Figure 11 shows one way of carrying out the procedure referred to above by using a separable tube 42 which is pushed into place within the opening in the skirt 4, the flexible construction of the skirt enabling the tube to be pushed into place with a tight sliding fit. If desired a depth stop abutment ring 43 can be provided to control the insertion of the tube into the skirt, as shown in Figure 11. Due to the deformities of the natural acetabulum this abutment ring 43 will not necessarily contact the skirt around the full periphery. if desired the abutment ring 43 could be a tight sliding fit on the tube 42 so that it could be moved under pressure on the tube to provide a further adjustable depth stop.

The lower end of the tube can have an abutment to provide stop means, or an arrangement as shown in Figure 2 can be used. The tube can be cut away once the cup has been inserted or can be simply dismantled from the cup and skirt.

If desired a step of the kind shown in Figures 5 and 6 can be provided and a similar technique employed.

In Figure 12 another method of employing the apparatus for implanting the cup 2 is shown and the same reference numerals as those used in Figure 3 are again used to indicate similar parts. With this method no skirt 4 is used.

The surgeon first prepares the socket 12 in the usual way and then employs a trepanning cutter in the manner shown in Figures 5 and 6 to provide a step 21 inside the natural bony rim. The outer wall 50 of the step 21 is dimensioned so that the outer wall 51 of the tube 7 is a push fit onto the step 21.

In order to implant the cup the surgeon first places the tube 7 in position on the step 21 and the cement is then pushed into place by manual operation of the piston 11. The piston is removed when the cement is partially set and in an adequately doughy stage. The cup is now immediately inserted down the tube 7 and the cement is pressurised from the moment the cup touches it. The cup is pushed further downwards into the socket 12 to the desired position provided by the adjustable disc 17.

A tube and piston construction as shown in Figure 1 can alternatively be used in a similar manner.

An adjustable tube 7 can be used, for example, of the kind shown in Figure 1.

Once again it will be seen from the drawing that no spacers are required in the cement mantle.

If required a skirt of the kind shown in Figure 1 could be used with this method to assist sealing.

With the methods of employing the apparatus shown in Figures 7, 8 and 11 the piston 11 can be released as described with regard to Figure 3 to remove the effect of the stop and allow the piston to be used to remove the tube.

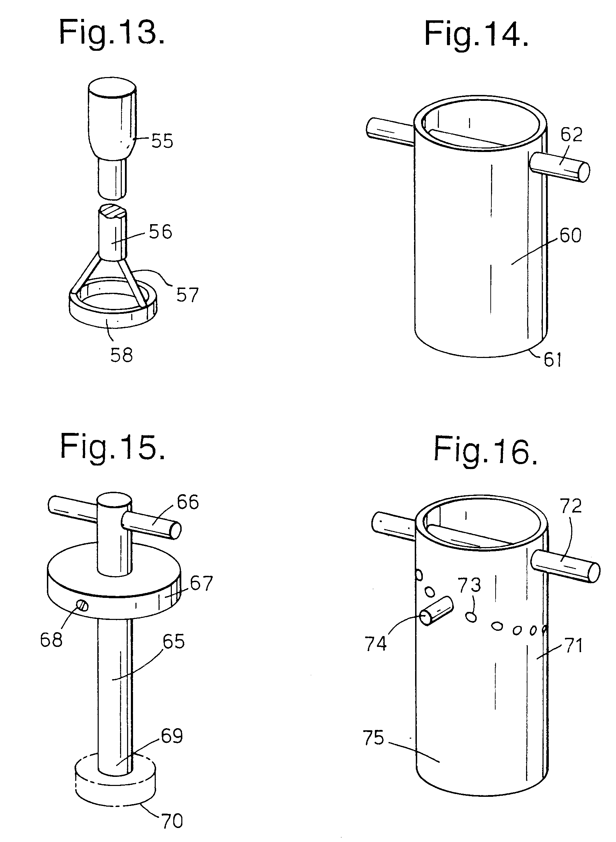

Figure 13 shows an alternative form of inserter which can be used for pushing the cup 2 into place. This inserter comprises a handle 55 secured to a rod 56 having a splayed end 57 secured to a ring 58. The diameter of the ring 58 is equivalent to the diameter of the annular flange 9 of the acetabular cup 2 with which it is to be used so that it acts on the rim of the cup 2 and this inserter is therefore employed with the construction shown in Figure 1. The cup is pushed down the tube until the peripheral portion of the edge of the front face of the ring 58 engages the abutment 8.

Figure 14 shows another alternative construction, again for use in the construction shown in Figure 1, and which comprises a tube 60, the outer diameter of which is similar to the inner diameter of the bore of the tube 7, and which thus provides an end surface 61 which can be of substantially the same diameter as the annular flange 9 on the cup 2.

A handle 62 is provided and the inserter is used in a similar way to that described with regard to Figure 13.

Figure 15 shows an alternative form of inserter which can be used with the type of construction shown in figures 3 and 12 and consists of an elongate rod 65 the upper end of which is provided with a handle 66 and which carries a disc 67 similar to the disc shown in Figure 1. The disc is again held by a grub screw 68 and the inserter is employed in a similar manner, the lower end 69 of the inserter resting against the inner bearing surface of the cup 2.

If desired a button, as shown in broken lines 7, can be provided on the end 69 to spread the load over the cup surface.

Figure 16 shows yet another alternative construction of inserter, again for use in the type of construction shown in Figure 3 and which comprises a tube 71 provided with a handle 72. The tube carries a descending ring of screw threaded holes 73 into which a screw threaded abutment 74 can alternatively be engaged. Thus the rising ring of holes 73 provide different height positions on the length of the tube 71.

The lower end 75 of the tube is of similar diameter to the annular flange 9 on the cup 2 so that when the inserter is used the lower end 75 pushed the cup down the tube until the adjustable abutment 74 engages the upper end of the tube, in a similar manner to the way the disc 67 engages the upper end of the tube as with the embodiment shown in Figure 15.

In order to allow further downward movement of the tube 71 after the cement has set the abutment 74 can be removed.

It will be appreciated that variations of the various embodiments described can be made by incorporating the different details and the method may vary by incorporating the different methods described together.

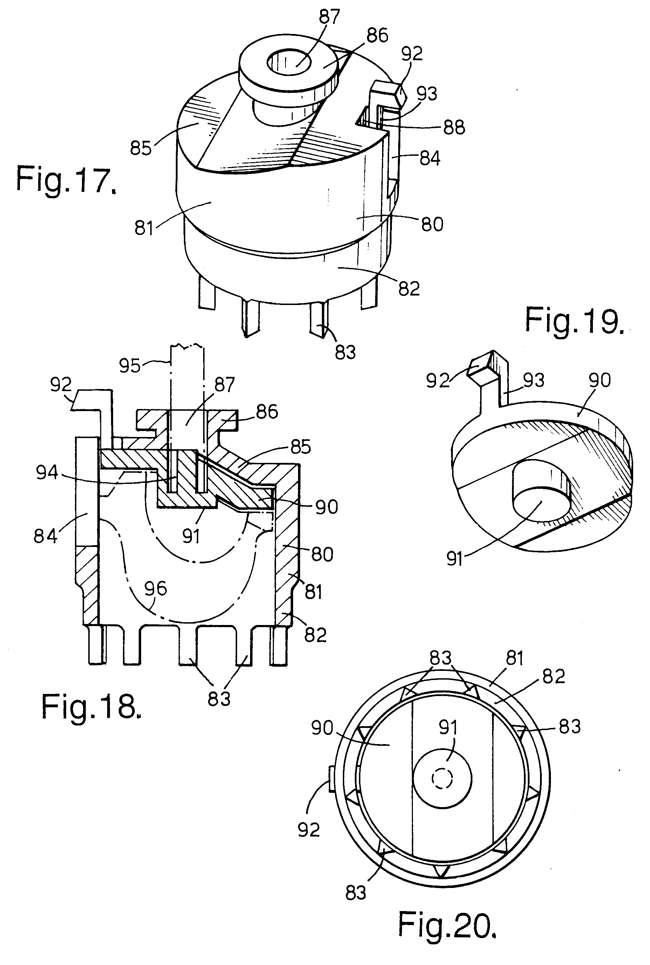

Figures 17 to 20 show another tube and inserter construction. In this arrangement the tube 80 has an upper portion 81 and a lower portion 82 of reduced diameter. The lower end of the portion 82 is castellated to provide a series of prongs 83 which are of triangular cross-section as is most clearly shown in Figure 20. The upper end 81 of the tube 80 is provided with a downwardly extending slot 84 and an upper end cover 85 which carries a boss 86 provided with a bore 87. the upper end cover 85 is also provided with a slot 88.

Located within the bore of the tube 80 is an inserter 90 and which comprises a shaped circular disc. The lower end of the disc carries a boss 91 and a flexible catch 92 is provided at one side.

The disc is made from a synthetic plastics material and the stem 93 of the catch is dimensioned so that it can resiliently bend rearwardly under pressure. The upper side of the disc is formed with an annular groove 94 dimensioned to receive a tubular push rod indicated by chain lines 95 in Figure 18.

The tube 80 is assembled with the inserter 90 in place within it and the catch 92 aligned in the slots 84 and 88. In Figure 18 a cup for insertion is indicated by chain lines 96.

When the construction shown in Figures 17 to 20 is to be used the socket to be fitted is first prepared in the manner described with regard to Figure 12, that is a cutter is used to provide a step 21 inside the natural bony rim. The outer diameter of the lower portion 82 of the tube 80 is dimensioned so that it is a push fit into the outer wall 50 of the step 21 and the depth of the step is arranged in relation to the lower end of the slot 84 of the tube so that the cup 96 is inserted with an adequate cement mantle. The lower end 82 of the tube 80 engages the lower end of the step 21 and it is for this reason that the castellated construction is provided which ensures that this lower end is of minimum cross-section. Thus the only portion of the lower end of the tube which engages the lower end of the step are the small cross-section prongs 83. This ensures that when the cement is applied to the socket there is only a minimum area of exposed bone around the rim of the cup. Due to this minimum cross-sectional area of the prongs 83 there is cement between the outer rim of the cup and the inner rim of the step 21.

The use of a sealing skirt may also be required especially in patients with extreme deformities.

It will be appreciated the depth of the rim cut by the trepanning cutter shown in Figures 3 and 4 can be controlled relative to the floor of the socket so that the eventual position of the tube and the cup and cement mantle thickness can be guaranteed with the correct use of the stop.

In the arrangement shown in figures 17 to 20 the cement can be applied to the socket before the tube is located in place or alternatively cement can be applied to the cup itself after it has been loaded into place in the tube.

After the tube has been located the push rod 95 is operated which causes the inserter 90 to descend thus pushing the cup into place. Pushing is continued until the stop, in the form of a catch 92, engages the lower end of the slot 84. This position is held until the cement has set. In order to now remove the tube from the cemented cup further pressure is applied to the push rod 95 which allows the catch 92 to be forced backwards against its resilient stem 93 thus allowing the inserter 90 to descend further to assist in the removal of the tube 80.

In the embodiment shown it will be seen that the inserter 90 is of a shape designed to engage a cup with a shaped rim. If the device is to be used with a straight rim then an appropriate inserter 90 of associated shape can be used.

The use of stops are helpful, as, when cementing a socket it is undesirable for the cup to break through the cement and touch the bone wall of a socket. Indeed, it is seen as a requirement of this product that the cement mantle should be continuous and uniform especially over the hemispherical area of the cup.

The essence of the present invention is to provide continuous cement pressurisation during the fitting of the socket and accurate location of the cup in the acetabular socket.

## Claims

1. Apparatus for implanting an acetabular cup characterised by a loading tube (7) adapted for alignment with an acetabular socket (1), the bove of said tube 7 being dimensioned to receive a suitably dimensioned acetabular cup (2) as a sliding fit to guide it into said socket (1), an inserter (11) for pushing said acetabular cup (2) down said tube (7), and stop means (8) (17) which act on said inserter (11) or an attachment (18) thereto to limit movement of said inserter (11) down said tube (7) to a predetermined position therein.

2. Apparatus as claimed in claim 1 characterised in that said inserter (11) acts on the rim of the cup (2).

3. Apparatus as claimed in claim 2 characterised in that said inserter (11) is in the form of a piston which is a sliding fit within the bore of the tube (7).

4. Apparatus as claimed in any one of preceding claims 1, 2 or 3 characterised in that said stop means (8) are provided on part of the bore of said tube (7) and act on the inner end of the inserter (11).

5. Apparatus as claimed in any one of preceding claims 1, 2 or 3 characterised in that said stop means (17) are provided on the inserter (11) and engage an abutment on said tube (7) located at a position paced away from the end of the tube (7) which is intended to be adjacent the acetabular socket (12) when in use.

6. Apparatus as claimed in any one of preceding claims 1 to 5 characterised in that the diameter of said tube (7) is adjustable.

7. Apparatus as claimed in any one of preceding claims 1 to 6 characterised in that said tube (7) is provided with a flexible skirt (4).

8. Apparatus as claimed in claim 7 characterised in that said skirt (4) is detachable from said tube (7).

9. Apparatus as claimed in claim 7 or claim 8 characterised in that said skirt (4) is provided with a cement escape control orifice (13).

10. Apparatus as claimed in any one of claims 7, 8 or 9 characterised in that said skirt (4) is corrugated (5).

11. Apparatus as claimed in any one of the preceding claims characterised in that one end of said tube (7) has a wedge or slope shaped orientation (A).

12. Apparatus as claimed in any one of the preceding claims characterised in that one end of said tube (7) is deformable.

13. Apparatus as claimed in any one of the preceding claims characterised by means (30) for orientating the tube (7) and/or skirt (4) on the bone into which a cup (2) is to be inserted.

14. Apparatus as claimed in any one of preceding claims 1 to 13 characterised in that said tube (7) and/or skirt (4) are made from a synthetic plastics material.

15. Apparatus as claimed in claim 14 characterised in that said skirt (4) is made from polyethylene, polypropylene or polyurethane.

16. Apparatus as claimed in claim 16 characterised in that said skirt (4) is made from a resorbable material.

17. Apparatus as claimed in claim 16 characterised in that said skirt (4) is made from polyglycolic acid or polylactic acid.

18. Apparatus as claimed in any one of preceding claims characterised by including means (19) for adjusting the position of said stop means (17).

19. Apparatus as claimed in any one of preceding claims 1 to 18 characterised in that said stop means (17) can be adjusted removed or overridden to allow said inserter (11) to be moved further down said tube beyond said predetermined position.

20. Apparatus as claimed in claim 19 characterised in that said stop means is provided by a resiliently biased catch (92) which can engage in a slot (84) provided in the wall of said tube (80) and which can move to an inoperative position to allow said inserter (90) to pass beyond a predetermined position.

21. Apparatus as claimed in any one of the preceding claims characterised in that a lower portion of the tube (7)(80) is of reduced external diameter.

22. Apparatus as claimed in any one of the preceding claims characterised in that the lower end of said tube (7)(80) which is adjacent the socket when in use is castellated (83).

## Patentansprüche

1. Vorrichtung zum Implantieren einer Hüftgelenkspfanne, gekennzeichnet durch ein zur Ausrichtung mit einer Hüftpfanne (1) angepaßtes Beschickungsrohr (7), wobei die Bohrung des besagten Rohrs (7) dimensioniert ist, um eine passend dimensionierte Hüftgelenkspfanne (2) in einer Gleitpassung aufzunehmen, um sie in die besagte Pfanne (1) zu führen, eine Einführvorrichtung (11), um die besagte Hüftgelenkspfanne (2) das besagte Rohr (7) hinabzudrücken, und Anschlageinrichtungen (8)(17), welche auf die besagte Einführvorrichtung (11) oder eine Zusatzeinrichtung (18) zu dieser einwirken, um eine Bewegung der besagten Einführvorrichtung (11) das besagte Rohr (7) hinab bis zu einer vorbestimmten Position darin zu begrenzen.

2. Vorrichtung nach Anspruch 1, dadurch gekennzeichnet, daß die besagte Einführvorrichtung (11) auf den Rand der Pfanne (2) einwirkt.

3. Vorrichtung nach Anspruch 2, dadurch gekennzeichnet, daß die besagte Einführvorrichtung (11) in Form eines Kolbens vorliegt, der eine Gleitpassung innerhalb der Bohrung des Rohrs (7) aufweist.

4. Vorrichtung nach einem beliebigen der vorangehenden Ansprüche 1, 2 oder 3, dadurch gekennzeichnet, daß die besagten Anschlageinrichtungen (8) auf einem Teil der Bohrung des besagten Rohrs (7) vorgesehen sind und auf das innere Ende der Einführvorrichtung (11) einwirken.

5. Vorrichtung nach einem beliebigen der vorangehenden Ansprüche 1, 2 oder 3, dadurch gekennzeichnet, daß die besagten Anschlageinrichtungen (17) auf der Einführvorrichtung (11) vorgesehen sind und mit einem Widerlager auf dem besagten Rohr (7) in Eingriff treten, das an einer Position im Abstand von demjenigen Ende des Rohrs (7) angeordnet ist, welches beim Gebrauch der Hüftpfanne (12) benachbart sein soll.

6. Vorrichtung nach einem beliebigen der vorangehenden Ansprüche 1 bis 5, dadurch gekennzeichnet, daß der Durchmesser des besagten Rohrs (7) verstellbar ist.

7. Vorrichtung nach einem beliebigen der vorangehenden Ansprüche 1 bis 6, dadurch gekennzeichnet, daß das besagte Rohr (7) mit einer flexiblen Schürze (4) versehen ist.

8. Vorrichtung nach Anspruch 7, dadurch gekennzeichnet, daß die besagte Schürze (4) von dem besagten Rohr (7) abnehmbar ist.

9. Vorrichtung nach Anspruch 7 oder Anspruch 8, dadurch gekennzeichnet, daß die besagte Schürze (4) mit einer Zementaustritts-Steueröffnung (13) versehen ist.

10. Vorrichtung nach einem beliebigen der Ansprüche 7, 8 oder 9, dadurch gekennzeichnet, daß die besagte Schürze (4) gewellt (5) ist.

11. Vorrichtung nach einem beliebigen der vorangehenden Ansprüche, dadurch gekennzeichnet, daß ein Ende des besagten Rohrs (7) eine keilförmige oder schräge Ausrichtung (A) aufweist.

12. Vorrichtung nach einem beliebigen der vorangehenden Ansprüche, dadurch gekennzeichnet, daß ein Ende des besagten Rohrs (7) verformbar ist.

13. Vorrichtung nach einem beliebigen der vorangehenden Ansprüche, gekennzeichnet durch Einrichtungen (30) zum Ausrichten des Rohrs (7) und/oder der Schürze (4) auf dem Knochen, in den eine Pfanne (2) eingesetzt werden soll.

14. Vorrichtung nach einem beliebigen der vorangehenden Ansprüche 1 bis 13, dadurch gekennzeichnet, daß das besagte Rohr (7) und/oder die Schürze (4) aus einem synthetischen Kunststoffmaterial hergestellt sind.

15. Vorrichtung nach Anspruch 14, dadurch gekennzeichnet, daß die besagte Schürze (4) aus Polyethylen, Polypropylen oder Polyurethan hergestellt ist.

16. Vorrichtung nach Anspruch 14, dadurch gekennzeichnet, daß die besagte Schürze (4) aus einem resorbierbaren Material hergestellt ist.

17. Vorrichtung nach Anspruch 16, dadurch gekennzeichnet, daß die besagte Schürze (4) aus Polyglykolsäure oder Polymilchsäure hergestellt ist.

18. Vorrichtung nach einem beliebigen der vorangehenden Ansprüche, dadurch gekennzeichnet, daß sie Einrichtungen (19) zum Verstellen der Position der besagten Anschlageinrichtungen (17) einschließt.

19. Vorrichtung nach einem beliebigen der vorangehenden Ansprüche 1 bis 18, dadurch gekennzeichnet, daß die besagten Anschlageinrichtungen (17) verstellt, entfernt oder überfahren werden können, um es zu ermöglichen, die besagte Einführvorrichtung (11) über die besagte vorbestimmte Position hinaus weiter das besagte Rohr hinabzubewegen.

20. Vorrichtung nach Anspruch 19, dadurch gekennzeichnet, daß die besagte Anschlageinrichtung von einer elastisch vorgespannten Sperre (92) bereitgestellt wird, die in einen in der Wand des besagten Rohrs (80) vorgesehenen Schlitz (84) eingreifen kann und die sich in eine nichtwirkende Position bewegen kann, um es zu ermöglichen, daß sich die besagte Einführvorrichtung (90) über eine vorbestimmte Position hinausbewegt.

21. Vorrichtung nach einem beliebigen der vorangehenden Ansprüche, dadurch gekennzeichnet, daß ein unterer Teil des Rohrs (7)(80) von kleinerem Außendurchmesser ist.

22. Vorrichtung nach einem beliebigen der vorangehenden Ansprüche, dadurch gekennzeichnet, daß das untere Ende des besagten Rohrs (7)(80), welches im Gebrauch der Pfanne benachbart ist, kronenförmig (83) ist.

## Revendications

1. Appareil pour l'implantation d'une cupule acétabulaire caractérisé en ce qu'un tube de mise en place (7) adapté pour être aligné avec une alvéole acétabulaire (1) l'alésage dudit tube (7) étant dimensionné pour recevoir une cupule acétabulaire de dimension adaptée (2) sous forme d'un agencement coulissant pour la guider jusqu'à l'intérieur de ladite alvéole (1), un dispositif d'insertion (11) pour pousser ladite cupule acétabulaire (2) vers le bas dudit tube (7), et des moyens d'arrêt (8) (17) qui agissent sur ledit dispositif d'insertion (11) ou sur un accessoire (18) de celui-ci pour limiter le mouvement dudit dispositif d'insertion (11) vers le bas dudit tube (7) à une position prédéterminée à l'intérieur de celui-ci.

2. Appareil selon la revendication 1, caractérisé en ce que ledit dispositif d'insertion (11) agit sur le bord de la cupule (2).

3. Appareil selon la revendication 2, caractérisé en ce que le dispositif d'insertion (11) a la forme d'un piston qui est agencé coulissant à l'intérieur de l'alésage du tube (7).

4. Appareil selon l'une quelconque des revendications 1, 2 ou 3, caractérisé en ce que lesdits moyens d'arrêt (8) sont agencés sur une partie de l'alésage dudit tube (7) et agissent sur l'extrémité intérieure du dispositif d'insertion (11).

5. Appareil selon l'une quelconque des revendications 1, 2 ou 3 caractérisé en ce que lesdits moyens d'arrêt (17) sont agencés sur le dispositif d'insertion (11) et viennent en contact avec une butée dudit tube (7) située au niveau d'une position espacée de l'extrémité du tube (7) qui est prévue pour être adjacente à l'alvéole acétabulaire (12) lorsqu'il est utilise.

6. Appareil selon l'une quelconque des revendications 1 à 5 caractérisé en ce que le diamètre dudit tube (7) peut être ajusté.

7. Appareil selon l'une quelconque des revendications 1 à 6, caractérisé en ce que ledit tube (7) est muni d'une collerette souple (4).

8. Appareil selon la revendication 7 caractérisé en ce que ladite collerette (4) est détachable dudit tube (7).

9. Appareil selon la revendication 7 ou 8 caractérisé en ce que ladite collerette (4) est munie d'un orifice (13) de commande d'échappement de ciment.

10. Appareil selon l'une quelconque des revendications 7, 8 ou 9 caractérisé en ce que ladite collerette (4) est ondulée (5).

11. Appareil selon l'une quelconque des revendications précédentes, caractérisé en ce qu'une première extrémité dudit tube (7) a une orientation (A) inclinée ou en forme de coin.

12. Appareil selon l'une quelconque des revendications précédentes, caractérisé en ce qu'une première extrémité dudit tube (7) est déformable.

13. Appareil selon l'une quelconque des revendications précédentes, caractérisé en ce qu'il comporte des moyens (30) pour orienter le tube (7) et/ou la collerette (4) sur l'os à l'intérieur duquel une cupule (2) doit être insérée.

14. Appareil selon l'une quelconque des revendications 1 à 13, caractérisé en ce que ledit tube (7) et/ou la collerette (4) sont constitués d'une matière plastique synthétique.

15. Appareil selon la revendication 14, caractérisé en ce que ladite collerette (4) est constituée de polyéthylène, de polypropylène ou de polyuréthanne.

16. Appareil selon la revendication 15, caractérisé en ce que ladite collerette (4) est constituée d'un matériau pouvant être résorbé.

17. Appareil selon la revendication 16, caractérisé en ce que ladite collerette (4) est constituée d'acide polyglycolique ou d'acide polylactique.

18. Appareil selon l'une quelconque des revendications précédentes, caractérisé en ce qu'il comporte des moyens (19) pour ajuster la position desdits moyens d'arrêt (17).

19. Appareil selon l'une quelconque des revendications 1 à 18, caractérisé en ce que lesdits moyens d'arrêt (17) peuvent être ajustés, enlevés ou surpassés pour permettre audit dispositif d'insertion (11) d'être déplacé plus vers le bas dudit tube au delà de ladite position prédéterminée.

20. Appareil selon la revendication 19, caractérisé en ce que lesdits moyens d'arrêt sont fournis par un cliquet rappelé élastiquement (92) qui peut venir en prise dans une fente (84) agencée dans la paroi dudit tube (80) et qui peut se déplacer vers une position inactive pour permettre audit dispositif d'insertion (90) de passer au delà d'une position prédéterminée.

21. Appareil selon l'une quelconque des revendications précédentes, caractérisé en ce qu'une partie inférieure du tube (7) (80) a un diamètre extérieur réduit.

22. Appareil selon l'une quelconque des revendications précédentes, caractérisé en ce que l'extrémité inférieure dudit tube (7) (80) qui est adjacente à l'alvéole lors de l'utilisation est crénelée (83).
